# EUROPEAN PATENT APPLICATION

(11) **EP 1 489 417 A1**
(43) Date of publication of application: **22.12.2004**
(21) Application number: 03712983.0
(22) Date of filing: 27.03.2003
(51) Int. Cl.: G01N 33/53, C12Q 1/00, C12M 1/00, C12N 15/00

(54) **TEST PIECE FOR ANALYZING SUBSTANCE WITH BIOLOGICAL ORIGIN, PROCESS FOR PRODUCING THE SAME AND METHOD OF EXAMINING TEST PIECE FOR SUBSTANCE WITH BIOLOGICAL ORIGIN**

(30) Priority: 28.03.2002 JP 2002091570; 07.01.2003 JP 2003001111
(71) Applicant: Olympus Corporation, Tokyo 151-0072 (JP)
(72) Inventor: AKIMOTO, Yoshinobu, Kokubunji-shi, Tokyo 185-0032 (JP); FUKUOKA, Morinao, Sagamihara-shi, Kanagawa 229-0003 (JP)
(74) Representative: von Hellfeld, Axel, Dr. Dipl.-Phys.
(86) International application number: PCT/JP2003/003853
(87) International publication number: WO 2003/083474

(57) **Abstract**

In a manufacturing method for a labeled test piece for analyzing an organism-oriented substance comprising a step for supplying a solution containing a specific binding substance with respect to an organism-oriented substance on a carrier, and a step for fixing the specific binding substance at a predetermined position on the carrier, the solution contains a detection substance differing from or identical to the label, which is dissolved or evenly dispersed independently of the specific binding substance. An examination method for a test piece for an organism-oriented substance wherein a specific binding substance with respect to the organism-oriented substance is fixed in a specific position on a carrier comprises, a step for supplying a labeled examination substance onto the carrier and fixing it in a different position from that of the specific binding substance, and a step for removing any unfixed examination substance.

## Description

### TECHNICAL FIELD

The present invention relates to a test piece for analyzing an organism-oriented substance, which can be used to analyze an organism-oriented substance such as genes, a manufacturing method therefor, and an examination method for a test piece for an organism-oriented substance.

### BACKGROUND ART

The method for analyzing genetic information can be classified into two main groups. One is to analyze "what" are the genes themselves, and mRNA and proteins expressed from the genes. The other is to analyze "under what conditions" the mRNA and proteins are expressed. The former method includes Western blotting, Northern blotting, and Southern blotting, which are mainly for analyzing observed proteins, DNA, or RNA.

On the other hand, the latter method includes the analysis of the interaction of transcription factors, signal transduction, and the like. However, this also aims to analyze observed single genes, and it is difficult to comprehensively analyze all expressed gene clusters in cells at once at a certain point in time.

Recently, techniques for fixing arbitrary oligonucleotides in high density on the surface of an about 1cm² carrier called a DNA chip and a DNA micro array chip have been developed, enabling all expressed gene clusters in cells to be comprehensively analyzed at once at a certain point in time.

A DNA chip is formed by dividing a silicon chip into a plurality of sections using photolithography and directly synthesizing single-stranded DNA having a specific base sequence on each section. On the other hand, the DNA micro array chip, is a DNA array chip which has conventionally an amount of about 300µl of DNA or more spotted on a membrane, or has an amount of about 200pl spotted on a slide glass.

If the type or arrangement of nucleic acid on the chip or the micro array chip is appropriately modified for use in connection with a signal reader or a computer system, it becomes possible to use such a DNA chip and DNA micro array chip for various applications such as gene mutation analysis, polymorphism analysis, base sequence analysis, expression analysis, and the like.

The use of genetic analysis employing the DNA micro array chip has just begun, and there are various problems at the present time in the manufacture of the micro array chip and the detector therefor. For example, the micro array chip is manufactured by plotting oligo DNA or cDNA by a device called a spotter. The manufacturing method includes a contact printing method wherein oligo DNA or cDNA are arranged on a slide glass by a pin in direct contact with the slide glass, and a non-contact printing method wherein oligo DNA or cDNA are blotted on the slide glass using the inkjet technique.

In the contact printing method, the sample is arranged on a carrier by a pin for spotting the sample such as oligo DNA or cDNA in direct contact with the carrier being a slide glass or the like. On the other hand, in the non-contact printing method, the sample is spotted on a carrier using the inkjet technique used in the field of printing.

In either printing method, there may be problems in that the quantity is irregular between the respective spots on the carrier, the shape of the respective spots is irregular, and sometimes no sample can be arranged in specific spots. Moreover, the position of the spot may be displaced. Such problems cause quality problems in the DNA micro array chip products manufactured by these methods. That is, it is inappropriate to use such a defective micro array chip for analysis requiring quantitativity. In some cases, it may also provide unreliable results even in qualitative analysis. Furthermore, depending on the degree of the defect, the micro array chip can not be shipped as a product, causing a problem of decreasing the yield rate in manufacture. A degree of irregularity of several % to several tens % is considered unavoidable at the present time.

Taking these problems into consideration, in Japanese Unexamined Patent Application, First Publication No. 2000-235036, there is proposed a test piece comprising a carrier on which a plurality of known specific binding substances differing respectively are respectively arranged at a plurality of predetermined positions, for analyzing an organism-oriented substance labeled with a labeling substance, wherein the specific binding substances are labeled with a labeling substance. Also proposed is a quantitative method for the organism-oriented substance using this test piece.

However, in this method, due to labeling (fluorescent labeling or the like) for detecting the amount of the specific binding substances arranged on the carrier, there are problems of increased cost, complicated manufacturing processes, and the like. For example, in the case where oligo DNA is arranged on the carrier as the specific binding substance, then in order to bind the oligo DNA to the carrier, it is not only necessary to add a labeling substance such as -NH₂ or -SH to the oligo DNA, but it is also required to label for detecting the oligo DNA itself. Therefore, in this method, two types of labeling are carried out with respect to one oligo DNA. Such problems become more remarkable if the DNA micro array chip is used where many types of specific binding substances are arranged on the carrier.

Moreover, there is another problem in that, if a plurality of types of labeling are used, the position for binding to the substance to be labeled is limited according to the type of the labeling, so that the type of the labeling substance and the labeling position are limited. That is, if fluorochrome such as Cy3 or Cy5 is used, then only the 5' terminal of the nucleic acid can be labeled, imposing a limitation such that the 3' terminal must be used for labeling the specific binding substance to bind to the carrier. Therefore, in the case described in Japanese Unexamined Patent Application, First Publication No. 2000-235036, the combinations of labeling is limited, which limits the degree of freedom in the design of the DNA micro array chip system.

Furthermore, in order to compare the detection result for the amount of signal emitted from the labeling of the specific binding substance, and the detection result for the amount of signal emitted from the labeling of the organism-oriented substance, it is necessary to use the different types of labeling substances for each, thus limiting the selection of the respective labeling substances.

There are not only such problems caused by the printing method, but also another problem in that, as disclosed in Japanese Unexamined Patent Application, First Publication No. Hei 11-187900, it is difficult to completely fix the oligo DNA or cDNA in terms of chemical reaction, and actual hybridization is the only way to ensure whether or not the oligo DNA or cDNA is fixed onto the carrier.

Therefore, from the point of cost and simplicity of the manufacturing process, a method for easily manufacturing a test piece for an organism-oriented substance at lower cost with a high degree of freedom in the design, and enabling easy examination of the test piece, is desired.

Methods for solving such problems include a method for improving the spotter so as to significantly reduce the irregularity between the spots, and a method for identifying material defects of the carrier which negatively affect fixation.

If an attempt is made to improve the spotter itself, it is necessary to arrange an amount of liquid in units of several pl to several hundreds pl, accurately and with high repeatability on the carrier, which is however naturally limited.

Therefore, an object of the present invention is to provide a manufacturing method for a test piece for analyzing an organism-oriented substance wherein it is possible to detect problems such as irregularity in the quantity between the spots, irregularity in the shape of the spots, and displacement of the position of the spots, even if these problems arise, without causing problems of greatly increasing the cost or complicating the manufacturing processes, and a test piece for analyzing an organism-oriented substances obtained by the method.

Furthermore, the present invention addresses the inferior quality of the carrier which is one factor to inhibit the fixation, with the object of providing an examination method including; a step for fixing the labeled examination substance separately from the specific binding substance onto the carrier and removing the unfixed examination substance from the carrier so as to easily ensure whether or not the carrier is suitable for manufacturing the test piece for an organism-oriented substance, and providing a manufacturing method for a test piece for analyzing an organism-oriented substance including this examination method, and a test piece for analyzing an organism-oriented substance obtained by the method.

### DISCLOSURE OF INVENTION

In order to solve the above problems and achieve the objects, the manufacturing method for a test piece for an organism-oriented substance of the present invention is constituted as below.

That is, the manufacturing method for a test piece for an organism-oriented substance of the present invention is characterized in that, in a manufacturing method for a test piece for analyzing an organism-oriented substance to which a label is attached, comprising a step for supplying a solution containing a specific binding substance with respect to an organism-oriented substance on a carrier, and a step for fixing the specific binding substance at a predetermined position; the solution contains a detection substance differing from or identical to the label, which is dissolved or evenly dispersed independently of the specific binding substance.

The manufacturing method for a test piece for analyzing an organism-oriented substance preferably further comprise a step for detecting the detection substance after the step for supplying the solution, or the step for fixing the specific binding substance. In this case, it may further comprise a step for removing the detection substance from the carrier after the step for detecting the detection substance.

Moreover, in the manufacturing method for a test piece for analyzing an organism-oriented substance of the present invention, the step for detecting the detection substance is preferably a step for detecting at least one of a position, a shape, a number, and a concentration of the detection substance on the carrier.

In the manufacturing method for a test piece for analyzing an organism-oriented substance of the present invention, the detection substance preferably has a different spectroscopic property from the spectroscopic property peculiar to the organism-oriented substance, the specific binding substance, and compounds of the organism-oriented substance and the specific binding substance. Moreover in the manufacturing method for a test piece for analyzing an organism-oriented substance of the present invention, the spectroscopic property is preferably the absorbance. Furthermore the detection substance may be selected from a group consisting of ink, dye, and paint.

A manufacturing method for a test piece for analyzing an organism-oriented substance of the present invention further comprises; a carrier examination step comprising a step for supplying a labeled examination substance onto a carrier and fixing it in a different position from that of a specific binding substance, and a step for removing any unfixed examination substance. Furthermore, the manufacturing method for a test piece for analyzing an organism-oriented substance of the present invention preferably comprises a step for detecting a label-oriented signal of a fixed examination substance after the step for removing any unfixed examination substance.

The test piece for analyzing an organism-oriented substance of the present invention is manufactured by the manufacturing method for a test piece for analyzing an organism-oriented substance of the present invention. More specifically, this is a DNA chip or a DNA micro array chip wherein the specific binding substance with respect to the organism-oriented substance is DNA.

The examination method for a test piece for analyzing an organism-oriented substance of the present invention is an examination method for a test piece for an organism-oriented substance in which a specific binding substance with respect to the organism-oriented substance is fixed in a predetermined position on a carrier comprising; a step for supplying a labeled examination substance onto the carrier and fixing it in a different position from that of the specific binding substance, and a step for removing any unfixed examination substance. Moreover, the examination method for a test piece for an organism-oriented substance of the present invention preferably comprises a step for detecting a label-oriented signal of a fixed examination substance after the step for removing the unfixed examination substance.

Furthermore, the examination method for a test piece for analyzing an organism-oriented substance of the present invention is an examination method for a test piece for an organism-oriented substance in which a specific binding substance with respect to the organism-oriented substance is fixed in a specific position on a carrier, comprising; a step for supplying a mixture of a detection substance and a specific binding substance onto a carrier and fixing the specific binding substance in a predetermined position, a step for supplying a labeled examination substance onto the carrier and fixing it in a different specific position from the predetermined position in the previous fixing step, and a step for removing the specific binding substance, the examination substance, and the detection substance which were not fixed in the two fixing steps. Moreover, the examination method for a test piece for analyzing an organism-oriented substance of the present invention preferably comprises a step for detecting a signal of a remaining detection substance on the carrier and a label-oriented signal of a fixed examination substance, after the step for removing the unfixed specific binding substance, the examination substance, and the detection substance.

### BEST MODE FOR CARRYING OUT THE INVENTION

### (Definitions)

In the present invention, the following terms are used for the meanings defined hereunder.

"Organism-oriented substance" includes not only animal cells, plant cells, and microbial cells, but also substances oriented from viruses and the like which are unable to proliferate by themselves without parasiting with such cells, and specifically includes proteins, nucleic acids, and the like. The organism-oriented substance includes not only substances extracted or isolated directly from these cells in the natural form, but also includes these substances produced using genetic engineering methods, modified chemically, and synthesized chemically. More specifically, the organism-oriented substance includes a hormone, a tumor marker, an enzyme, an antibody, an antigen, abzymes, other proteins, a nucleic acid, cDNA, DNA, mRNA, and the like. In the present invention, the organism-oriented substance is labeled with a fluorescent substance or the like. However, the labeling substance and the labeling method are not specifically limited, provided they are known in the technical field, and are determined by considering the detection system used for the organism-oriented substance.

"Specific binding substance" means a substance bindable specifically with the above organism-oriented substance, specifically including a ligand such as a hormone and the receptor thereof, an enzyme and the substrate thereof, an antigen such as a tumor marker and the antibody thereof, a nucleic acid having a specific sequence and the nucleic acid having the complementary sequence thereof, and any substance having such a relation.

"Test piece" includes a plurality of spots designed to be in a fixed arrangement on the carrier and treated for enabling fixing of the substance on the surface of the respective spots, meaning a piece used for appropriately fixing the substance onto the spots, binding the substance and other substances bindable thereof on the respective spots, and for detecting the compounds formed of the substance and other substances. The test piece includes a DNA chip and a DNA micro array chip.

"Detection substance" is a different substance from the organism-oriented substance, meaning a substance having a different spectroscopic property from the spectroscopic property peculiar to the organism-oriented substance, the specific binding substances, and the compounds thereof.

"Examination substance" is a substance used for ensuring the quality of the carrier, and is usable if it binds in a similar style to the style between the specific binding substance and the carrier. Specifically, it may be a similar substance to the specific binding substance. However it is not necessary to bind specifically with the organism-oriented substance.

Hereunder is a description of the construction, the execution method, and the effects of embodiments of the present invention.

In first aspect, the manufacturing method for a test piece for an organism-oriented substance of the present invention is characterized in that in a manufacturing method for a test piece for analyzing an organism-oriented substance to which a label is attached; comprising a step for supplying a solution containing a specific binding substance with respect to the organism-oriented substance on a carrier, and a step for fixing the specific binding substance at a predetermined position, the solution contains a detection substance differing from or identical to the labeling, which is dissolved or evenly dispersed independently of the specific binding substance.

The solution containing the specific binding substance used in the present invention (or disperse system or suspension) also contains the detection substance differing from or identical to the labeling to be detected. This detection substance is dissolved, evenly dispersed or suspended into the solution independently of this specific binding substance. Being dissolved, dispersed or suspended independently means that the specific binding substance and the detection substance are not previously chemically bound, and are respectively contained as the separate solute or disperse phase evenly in the solution (dispersion or suspension). Furthermore, the detection substance is preferably ineffective in inhibiting binding of the specific binding substance and the organism-oriented substance.

The carrier used in the method of the present invention is not specifically limited as long as it is suitable for fixing the specific binding substance. However it may be a porous carrier, in addition to a carrier where the respective sections are provided as a plane such as with a slide glass, a silicon wafer, a membrane filter, and the like for example. The porous carrier includes for example an aluminium anode oxide film comprising multiply arranged minute liquid containing sections capable of containing the liquid in three dimensions. Here, the liquid containing section is the minimum unit for fixing the specific binding substance. Since such a porous carrier can fix a large amount of specific binding substance to one liquid containing section and can quantitatively fix the specific binding substance, it is suitable for quantitative experiments such as expression level analysis. Moreover, a porous carrier made by etching a silicon wafer may be applicable.

In the method of the present invention, since the labeling for detecting the amount of specific binding substance fixed onto the carrier is not bound directly with the specific binding substance, there are advantages in the cost, time and effort for preparing the specific binding substance, enabling preparation of the test piece easily at very low cost. Moreover, since the labeling to the specific binding substance requires only one type for fixing onto the carrier, the degree of freedom for selecting the labeling is high. Furthermore, since it can be confirmed that the specific binding substance is spotted on the carrier accurately according to the setting, the test piece prepared by this method can be used for quantitative analysis, and quality assurance of the product can be ensured.

The method of the present invention may further comprise a step for detecting the detection substance after supplying the abovementioned solution onto the carrier. That is, in the present invention, the detection substance may be detected at the manufacturing stage, as the quality inspection step. Moreover, the user may ensure the quality of the test piece before using it, without removing the detection substance during the manufacturing process.

If the method of the present invention comprises the step for detecting the detection substance, it may further comprise a step for removing the detection substance from the carrier after the step for detecting. The removing method is not specifically limited as long as it is a method for selectively removing the detection substance while the specific binding substance fixed onto the carrier is kept from being removed from the carrier. Specifically, it includes a method using a solvent having an identical composition to the solvent used in the solution containing the organism-oriented substance and the detection substance, and a solvent having a modified composition in the salt concentration or pH, but it is not limited to these. In the case where the detection substance can possibly affect the detection of the compound of the specific binding substance and the organism-oriented substance, the detection substance is preferably removed before using the test piece. However, when carrying out the method of the present invention, if a detection substance not affecting the detection of the organism-oriented substance is used, it is possible to omit the step for removing the detection substance after fixing the specific binding substance to the carrier. This is preferable from the point of simplifying the steps and eliminating the possibility that the specific binding substance is detached from the carrier during the removing step.

In the method of the present invention, in the step for detecting the detection substance, it is preferable to detect at least one of the position, shape, number, and concentration of the detection substance on the carrier. The data of the position, shape, and number of the detection substance on the carrier is useful for ensuring if they agree with the design of the test piece so as to ensure the quality of the test piece. Here, the shape of the spot includes ensuring the presence or absence of a satellite spot. If a solution is spotted on the carrier by the inkjet method, the droplets may not congregate into one droplet on the carrier, generating one larger droplet and at least one small droplets (caused by splashing) in the vicinity thereof. The satellite spot means such a droplet caused by splashing in the above case. If the satellite spot is generated in this manner, it is preferable not to use the spot when using the test piece.

On the other hand, if the calibration curve for the detection substance to be used is previously formed, the data for the concentration of the detection substance on the carrier is useful from the point of enabling checking and correcting of the amount of the specific binding substance actually spotted on the carrier of the test piece.

The spot of the detection substance on the test piece can be examined for example by capturing the image of the test piece using a CCD camera or the like, carrying out image processing, and ensuring that the result is compatible with preset data. This operation can be carried out in large quantities by appropriate use of a computer. Moreover, if the combination of data obtained from the examination, and the test piece used for the examination, are combined and controlled by a database, the quality of the amount of the specific binding substance on the test piece can be controlled. Furthermore, if the test piece is distributed into the market with a set of this data on the amount, the user can correct the data obtained by using the test piece. Therefore the test piece of the present invention can be used for experiments requiring higher accuracy in quantitativity.

The detection substance used in the present invention has a different spectroscopic property from the spectroscopic property peculiar to the organism-oriented substance, the specific binding substance, and the compounds thereof. The spectroscopic property is preferably the absorbance. The method according to and suitable for the spectroscopic property of the detection substance to be used, is used for the method for detecting the detection substance, for example including a method for measuring a wavelength peculiar to the detection substance such as the spectrum of visible-light, ultraviolet, and infrared zone. The case of measuring the spectrum of visible-light zone is preferable for reducing the cost of the detection system.

In the method of the present invention, the detection substance may be used by selecting from a group consisting of ink, dye, and paint. In more detail, it includes for example, fluorochrome (Cy3, Cy5, rhodamine, tamura, FITC or the like), carbon black, black oxide, red ocher, orange G, azo dye, phthalocyanine dye, fluorescent ink, fluorescent dye, and particles called quantum dots having a diameter of nm order such as gold, silver, silicon, and other various semiconductors. Water is often used for preparing the specific binding substance when printing the specific binding substance on the carrier. The detection substance is preferably water soluble, or water dispersible. Moreover, the detection substance is not bindable directly with the specific binding substance.

The amount of detection substance is not specifically limited as long as the detection substance can be dissolved, evenly dispersed or suspended into the solution containing the specific binding substance, and then detected in the steps thereafter. The amount is preferably enough not to inhibit the binding of the specific binding substance to the carrier, and the compound formation of the specific binding substance bound to the carrier and the specific organism-oriented substance.

The specific binding substance can be fixed onto the carrier according to the type of the specific binding substance and the property of the fixation surface of the carrier, by using any method known in the technical field related to the present invention. Examples include a method described in the reference of Biochem. Biophys. Res. Commun. [1] (1978) 1-6, Nucleic Acids Res. [16] (1988) 10861-10880, and the like.

Moreover, the present invention provides an examination method for a test piece for an organism-oriented substance comprising, a step for supplying a labeled examination substance onto a carrier to fix it in a different position from that of a specific binding substance, and a step for removing any unfixed examination substance. The labeled examination substance used in the step for supplying the labeled examination substance onto the carrier to fix it in the different position from that of the specific binding substance, means an examination substance which is labeled by being directly or indirectly bound with fluorescence, radioisotope, chemiluminescence, quantum dots, and the like by a known method, which can be properly detected and measured as a signal by an appropriate device. Moreover, the same type of specific binding substance can be used as the examination substance. For example, if oligo DNA is used as the specific binding substance, the oligo DNA may be used as the examination substance.

The method for fixing the labeled examination substance may be carried out according to the type of the examination substance and the property of the fixation surface of the carrier by using any method known in the technical field related to the present invention. Examples include a method similar to the method for fixing the specific binding substance onto the carrier.

In the step for removing the unfixed examination substance in the examination method of the present invention, any method may be used as long as the unfixed examination substance can be removed while the already-fixed specific binding substance and the fixed examination substance are kept from being removed from the carrier. Specifically, it includes a method using a solvent having an identical composition to the solvent used in the solution containing the specific binding substance and the examination substance, and a solvent having a modified composition in the salt concentration or pH, and a method using sterile water to wash the carrier and dry thereafter. At this time, the unfixed specific binding substance may be removed together with the unfixed examination substance.

After this step, the label-oriented signal of the fixed examination substance is detected. If the label-oriented signal of the examination substance is detected, the carrier can be judged as one appropriate for binding with the specific binding substance, and that is suitable for manufacturing the test piece. Conversely, if the label-oriented signal of the examination substance is not detected, the carrier can be judged as unsuitable for manufacturing the test piece due to inferior quality. Furthermore, this step may be carried out for the purpose of ensuring the quality of the test piece by the user before using it.

The present invention further provides an examination method for a test piece for an organism-oriented substance comprising, a step for supplying a mixture of a detection substance and a specific binding substance onto a carrier to fix the specific binding substance in a specific position, a step for supplying a labeled examination substance onto the carrier to fix it in a different specific position from the specific position in the fixing step of the specific binding substance, and a step for removing the specific binding substance, the examination substance, and the detection substance which were not fixed in these steps.

In the description of the invention according to this embodiment, description of the points capable of having a construction and method of execution similar to those in the other abovementioned embodiments is omitted.

The removing method in the step for removing the unfixed specific binding substance, the examination substance, and the detection substance in the invention according to the present embodiment, is not specifically limited as long as it is a method for selectively removing the unfixed specific binding substance and the examination substance while the fixed specific binding substance and the fixed examination substance are kept from being removed from the carrier, and removing the detection substance mixed with the specific binding substance and adhered thereto. Specifically, it includes a method using a solvent having an identical composition to the solvent used in the solution containing the specific binding substance and the examination substance, and a solvent having a modified composition for the salt concentration or pH, and a method using sterile water to wash the carrier and dry thereafter.

After this step, the signal of the remaining detection substance on the carrier and the label-oriented signal of the fixed examination substance are detected. If the signal of the detection substance is not detected and the label-oriented signal of the examination substance is detected, the carrier can be judged as one appropriate for binding with the specific binding substance, and that is suitable for manufacturing the test piece.

In the present embodiment, the detection substance becomes the index to determine whether or not the unfixed specific binding substance and examination substance are completely removed. That is, if the unfixed specific binding substance and examination substance are not completely removed in the step for removing the specific binding substance, the examination substance, and the detection substance, an error examination result would be inferred by the unfixed examination substance remaining on the carrier even if the carrier is actually inferior. This step may be carried out for the purpose of ensuring the quality of the test piece by the user before using it.

Moreover, the present invention also provides a test piece for an organism-oriented substance manufactured by the abovementioned manufacturing method. Compared to a test piece manufactured by the conventional method, the test piece manufactured by the method of the present invention is different in construction in that the test piece is checked to see if the specific binding substance is spotted in the predetermined position on the carrier according to the setting, or the test piece can be checked by the user immediately at the time of using it. Such difference has an advantage which the conventional techniques do not have, in the point that the test piece can be used for an examination requiring quantitativity, which has been impossible or inadequate with the conventional test piece. Moreover, since product defects based on the inferior quality of the carrier can be known without carrying out the hybridization reaction, wastage of the sample by the user can be prevented, and it becomes possible to predict that the specific binding substance is not fixed according to the design, due to the inferior carrier. By omitting these faulty products in the manufacturing process, the yield rate of manufacture is increased, enabling only products having superior quality to be shipped.

The test piece for an organism-oriented substance of the present invention includes a DNA chip and a DNA micro array chip. However it is applicable for examining nucleic acids other than DNA, and other organism-oriented substances such as proteins, by appropriately modifying the specific binding substance.

### Examples

### (Example 1)

A DNA micro array chip having a 76mm x 26mm x 1mm slide glass as a carrier with the surface pretreated with 1wt% of poly-L-lysine solution, was used. A plurality of different amino-labeled oligo DNAs having known base sequences were used as the specific binding substance. FITC (fluorescein isothiocyanate) of fluorochrome was used as the detection substance. The solution was prepared by dissolving both the specific binding substance and the detection substance therein. Using a spotter, 100pl of the solution of the specific binding substance and the detection substance was spotted in the predetermined position on the carrier of the DNA micro array chip, so as to make a DNA micro array chip having a specific binding substance fixed in predetermined positions on the carrier.

Using a fluorescence microscope having a CCD camera (BX-51 made by Olympus Co. Ltd.,), the size, the shape, the amount of fluorescence, and the arrangement of the respective spots on the DNA micro array chip were measured. Then it was confirmed by image analysis whether or not they were within the predetermined standard value.

All of the size, the shape, the amount of fluorescence, and the arrangement of the respective spots on the DNA micro array chip could be confirmed very easily by an image analyzing system using a fluorescence microscope.

### (Example 2)

In example 1, the size, the shape, the amount of fluorescence, and the arrangement of the respective spots on the DNA micro array chip were measured and it was confirmed that they were within the predetermined standard value. Then, the micro array chip made thereby was washed with 100ml of 0.2 x SSC solution three times to remove the FITC being the detection substance. Next, the nucleic acid of the organism-oriented substance was labeled with FITC and bound to the specific binding substance on the micro array chip. The organism-oriented substance could be detected with a better signal/noise ratio than that for the case where the organism-oriented substance was bound without washing the detection substance on the micro array chip.

### (Example 3)

Similarly to the operation of example 1, with an exception that carbon colloidal particles having a particle size of 0.5µm or less were dispersed as the detection substance, the fixation of the specific binding substance to the DNA micro array chip was examined.

Since a detection substance which was not fluorochrome but had a characteristic absorption spectrum in the visible-light zone was used, the detection was carried out in a simpler detection system than that of example 1. Moreover, since it was black, it was easier to binarize so that the image processing was also carried out more easily. In this manner, the non-fluorescent substance was used as the detection substance so that it was more advantageous in the detection of the organism-oriented substance labeled with fluorescent labeling.

### (Example 4)

100 types of known unlabeled oligo DNA and one type of known oligo DNA (examination oligo) having the 5' terminal labeled with FITC (fluorescein isothiocyanate) were fixed onto a slide glass coated with polycarbodiimide resin, in predetermined positions using an inkjet spotter.

This slide glass was washed sequentially with PBS buffer and sterile water so as to remove any unfixed oligo DNA from the slide glass. After drying the slide glass with a drier, the position expected to be fixed with the examination oligo was observed by a fluorescence microscope, and the presence of fluorescence was confirmed. Therefore, it was judged that the 100 types of known unlabeled oligo DNA were fixed in the predetermined positions according to the design.

Next, the labeled sample containing the DNA bindable specifically with the 100 types of oligo DNA was contacted with this slide glass, then washed, and detected, confirming excellent signals having sufficient intensity from all the 100 types of spots.

### (Example 5)

100 types of known unlabeled oligo DNA and one type of known oligo DNA (examination oligo) having the 5' terminal labeled with FITC (fluorescein isothiocyanate) were fixed onto a slide glass coated with poly-L-lysine, in predetermined positions using the inkjet spotter. This slide glass was washed sequentially by PBS buffer and sterile water so as to remove the unfixed oligo DNA from the slide glass. After drying the slide glass with a drier, the position expected to be fixed with the examination oligo was observed by the fluorescence microscope, and the presence of fluorescence was confirmed. Therefore, it was judged that the 100 types of known unlabeled oligo DNA were fixed in the predetermined positions according to the design.

Next, the labeled sample containing the DNA bindable specifically with the 100 types of oligo DNA was contacted with this slide glass, then washed, and detected, confirming excellent signals having sufficient intensity from all the 100 types of spots.

### (Example 6)

100 types of known oligo DNA having the amino-modified 5' terminal and mixed with Cy3 dye and one type of known oligo DNA (examination oligo) having the amino-modified 5' terminal and the 3' terminal labeled with FITC (fluorescein isothiocyanate) were fixed onto a slide glass having the surface modified with active aldehyde group, in predetermined positions using the inkjet spotter. Before washing, the position of the spot was confirmed by detecting the Cy3 dye. Then, this slide glass was washed sequentially with PBS buffer and sterile water so as to remove the unfixed oligo DNA from the slide glass. After drying the slide glass with a drier, the position expected to be fixed with the 100 types of oligo DNA was observed by the fluorescence microscope, but the presence of fluorescence of Cy3 was not confirmed. Therefore, it was judged that the unfixed oligo DNA mixed with the Cy3 dye and the unfixed examination oligo had been removed. Moreover, the position expected to be fixed with the examination oligo was observed, and the presence of fluorescence was confirmed. Therefore, it was judged that the 100 types of known oligo DNA were fixed in the predetermined positions according to the design.

Next, the labeled sample containing the DNA bindable specifically with the 100 types of oligo DNA was contacted with this slide glass, then washed, and detected, confirming excellent signals having sufficient intensity from all the 100 types of spots.

From the above results, on the test piece where fixation of one type of the examination oligo was confirmed, it was confirmed that the other DNA oligo was also surely fixed, from the results of the hybridization carried out thereafter.

### INDUSTRIAL APPLICABILITY

In the manufacturing method for a test piece for analyzing an organism-oriented substance of the present invention, since the solution contains the detection substance differing from or identical to the labeling of the labeled organism-oriented substance which is dissolved or evenly dispersed independently of the specific binding substance, the fixation of the specific binding substance to the test piece can be examined by a simple method at very low cost.

Moreover, since defective products caused by inferior carriers can be discovered and excluded in the manufacturing process, the yield rate of manufacture can be increased. Furthermore, wastage of the sample by the user, and erroneous examination results due to defects of the test piece can be prevented.

## Claims

1. A manufacturing method for a test piece for analyzing an organism-oriented substance to which a label is attached, comprising a step for supplying a solution containing a specific binding substance with respect to an organism-oriented substance on a carrier, and a step for fixing the specific binding substance at a predetermined position, wherein
said solution contains a detection substance differing from or identical to said label, which is dissolved or evenly dispersed independently of said specific binding substance.

2. A method according to claim 1, further comprising a step for detecting said detection substance after said step for supplying the solution, or said step for fixing the specific binding substance.

3. A method according to claim 2, further comprising a step for removing said detection substance from said carrier after said step for detecting the detection substance.

4. A method according to either one of claim 2 and claim 3, wherein said step for detecting the detection substance is a step for detecting at least one of a position, a shape, a number, and a concentration of said detection substance on said carrier.

5. A method according to any one of claim 1 through claim 4, wherein said detection substance has a different spectroscopic property from the spectroscopic property peculiar to said organism-oriented substance, said specific binding substance, and compounds of said organism-oriented substance and said specific binding substance.

6. A method according to claim 5, wherein said spectroscopic property is the absorbance.

7. A method according to any one of claim 1 through claim 6, wherein said detection substance is selected from a group consisting of ink, dye, paint and quantum dots.

8. A method according to any one of claim 1 through claim 7, comprising a carrier examination step comprising;
a step for supplying a labeled examination substance onto a carrier and fixing it in a different position from that of a specific binding substance, and
a step for removing any unfixed examination substance.

9. A method according to claim 8, comprising a step for detecting a label-oriented signal of a fixed examination substance, after said step for removing any unfixed examination substance.

10. A test piece for analyzing an organism-oriented substance, manufactured by the method according to any one of claim 1 through claim 9.

11. A test piece for analyzing an organism-oriented substance according to claim 10, wherein the specific binding substance with respect to said organism-oriented substance is DNA.

12. An examination method for a test piece for an organism-oriented substance in which a specific binding substance with respect to the organism-oriented substance is fixed in a predetermined position on a carrier comprising;
a step for supplying a labeled examination substance onto the carrier and fixing it in a different position from that of the specific binding substance, and
a step for removing any unfixed examination substance.

13. An examination method for a test piece for an organism-oriented substance according to claim 12, further comprising a step for detecting a label-oriented signal of a fixed examination substance, after said step for removing any unfixed examination substance.

14. An examination method for a test piece for an organism-oriented substance in which a specific binding substance with respect to the organism-oriented substance is fixed in a specific position on a carrier, comprising;
a step for supplying a mixture of a detection substance and a specific binding substance onto a carrier and fixing the specific binding substance in a predetermined position,
a step for supplying a labeled examination substance onto the carrier and fixing it in a different specific position from the predetermined position in the previous fixing step, and
a step for removing the specific binding substance, the examination substance, and the detection substance which were not fixed in said two fixing steps.

15. An examination method for a test piece for an organism-oriented substance according to claim 14, comprising a step for detecting a signal of a remaining detection substance on the carrier and a label-oriented signal of a fixed examination substance, after said step for removing the unfixed specific binding substance, the examination substance, and the detection substance.
